# EUROPEAN PATENT SPECIFICATION

(11) **EP 1 731 603 B1**
(45) Date of publication and mention of the grant of the patent: **09.05.2012**
(21) Application number: 05720845.6
(22) Date of filing: 09.03.2005
(51) Int. Cl.: C12N 15/09, C12Q 1/68, G01N 33/15, G01N 33/50

(54) **METHODS OF DETECTING AND QUANTIFYING PROTEIN NUCLEAR MIGRATION USING PAIRS OF PROBES**
VERFAHREN ZUM NACHWEIS UND ZUR QUANTIFIZIERUNG DES KERNTRANSPORTS VON PROTEINEN UNTER VERWENDUNG VON SONDENPAAREN
PROCEDE DE DETECTION ET DE QUANTIFICATION DU TRANSPORT NUCLEAIRE DE PROTEINES EN UTILISANT DES PAIRES DE SONDES

(30) Priority: 09.03.2004 JP 2004066424
(43) Date of publication of application: 13.12.2006
(73) Proprietor: Japan Science and Technology Agency, Kawaguchi-shi, Saitama 332-0012 (JP)
(72) Inventor: UMEZAWA, Yoshio, Shinjuku-ku, Tokyo 162-0063 (JP); OZAWA, Takeaki, Matsudo-shi, Chiba 270-2231 (JP)
(74) Representative: Owen, Deborah Jane
(86) International application number: PCT/JP2005/004591
(87) International publication number: WO 2005/085439

(56) References cited:
- WO-A-00/71701
- WO-A-2004/027057
- WO-A1-2004/104222
- US-A1- 2002 106 632
- KIM SUNG BAE ET AL: "High-throughput sensing and noninvasive imaging of protein nuclear transport by using reconstitution of split Renilla luciferase" PROCEEDINGS OF THE NATIONAL ACADEMY OF SCIENCES OF THE UNITED STATES OF AMERICA, vol. 101, no. 32, 2 August 2004 (2004-08-02), pages 11542-11547, XP002479375 ISSN: 0027-8424
- PAULMURUGAN R ET AL: "Monitoring protein-protein interactions using split synthetic Renilla luciferase protein-fragment-assisted complementation" ANALYTICAL CHEMISTRY, AMERICAN CHEMICAL SOCIETY. COLUMBUS, US, vol. 75, no. 7, 1 April 2003 (2003-04-01), pages 1584-1589, XP001170664 ISSN: 0003-2700
- HU C-D ET AL: "Visualization of interactions among bZIP and Rel family proteins in living cells using bimolecular fluorescence complementation" MOLECULAR CELL, CELL PRESS, CAMBRIDGE, MA, US, vol. 9, no. 4, 1 April 2002 (2002-04-01), pages 789-798, XP002253973 ISSN: 1097-2765
- DUNNWALD M ET AL: "Detection of transient in vivo interactions between substrate and transporter during protein translocation into the endoplasmic reticulum" MOLECULAR BIOLOGY OF THE CELL, BETHESDA, MD, US, vol. 10, no. 2, 1 February 1999 (1999-02-01), pages 329-344, XP008006622 ISSN: 1059-1524
- OZAWA T ET AL: "Detection of protein-protein interactions in vivo based on protein splicing" CURRENT OPINION IN CHEMICAL BIOLOGY, CURRENT BIOLOGY LTD, LONDON, GB, vol. 5, no. 5, 1 October 2001 (2001-10-01), pages 578-583, XP002377401 ISSN: 1367-5931
- VILLALOBOS VICTOR ET AL: "Current state of imaging protein-protein interactions in vivo with genetically encoded reporters" ANNUAL REVIEW OF BIOMEDICAL ENGINEERING, vol. 9, 2007, pages 321-349, XP002479376 ISSN: 1523-9829
- OZAWA T. ET AL: 'Split luciferase as an optical probe for detecting protein-protein interactions in mammalian cells based on protein splicing.' ANALYTICAL CHEMISTRY. vol. 73, no. 11, 2001, pages 2516 - 2521, XP001081130
- OZAWA T. ET AL: 'A fluorescent indicator for detecting protein-protein interactions in vivo based on protein splicing.' ANALYTICAL CHEMISTRY. vol. 72, no. 21, 2000, pages 5151 - 5157, XP002243310
- PAULMURUGAN R. ET AL: 'Noninvasive imaging of protein-protein interactions in living subjects by using reporter protein complementation and reconstruction strategies.' PRO NATL ACAD SCI USA. vol. 99, no. 24, 2002, pages 15608 - 15613, XP002980935
- KIM S.B. ET AL: 'Ikita Mouse Kotainai deno Dansei Hormone-yo Kagaku Busshitsu no Shigeki ni yoru Androgen Receptor no Kakunai Iko no Hishinshu Kashika Bunseki. (2 J6-08)' THE CHEMICAL SOCIETY OF JAPAN. vol. 84, no. 2, 11 March 2004, page 1115, XP002989525
- VANDROMME M ET AL.: "Two nuclear localization signals present in the basic-helix 1 domains of MyoD promote its active nuclear translocation and can function independently.", PROC NATL ACAD SCI USA, vol. 92, no. 10, 9 May 1995 (1995-05-09), pages 4646-4650,

## Description

### Technical Field

This application relates to a pair of probes for detecting and quantifying protein nuclear transport induced by action of bioactive substances. More specifically, the invention of this application relates to a method for detecting and quantifying protein nuclear transport using the same, and a method for screening substances which induce or inhibit protein nuclear transport.

### Background Art

The control of complicated signaling networks within eukaryotic cells relies on the compartmentalization of each protein. Nucleocytosolic trafficking of proteins in response to extra- or intracellular stimuli is an essential step for regulating the magnitude and specificity of gene expressions. The trafficking is regulated by posttranslational modifications of proteins, which include ligand-receptor binding, protein phosphorylation, and proteolysis.

Because various nuclear proteins are mislocalized in cancer cells, there is an intense interest in identifying small molecules that redirect the proteins to the correct compartments. Also, the nuclear localization of the proteins is altered in the cells that are exposed to specific exogenous chemicals, of which potential effects on living animals are the major concern.

Hence, development of a rapid screening system to detect the nucleocytosolic trafficking is essential for the discovery of novel compounds that have anticancer activity or for testing the toxicity of chemicals, from which new insights into the mechanism of nucleocytosolic trafficking could be provided (non-Parent Documents 1-4).

A technique for monitoring the dynamics of the protein movement inside single living cells relies on the use of immunocytochemistry or optical imaging with genetically tagged GFP (non-Patent Document 5). These analyses are effective for imaging the spatial and temporal dynamics of proteins of interest within single living cells. However, there have been problems that it takes a long time to confirm and quantify the intracellular localization under a microscope, and analyses of the protein localization in living animals require complex assay procedures such as extraction of an organ and dividing it into sliced sections.
non-Patent Document 1: Kau, T. R. & Silver, P. A. Drug Discov. Today 8, 78-85 (2003).
non-Patent Document 2: Kau, T. R. et al. Nat. Rev. Cancer 4, 1-12 (2004).
non-Patent Document 3: Rudin, M. & Weissleder, R. Nat. Rev. Drug Discov. 2, 123-13 1 (2003).
non-Patent Document 4: Gray, L. E., Jr. et al. Toxicology 181-182, 371-382 (2002).
non-Patent Document 5: Elion, E. A. Methods Enzymol. 351, 607-622 (2002).
non-Patent Document 6: Singh, S. M. et al. Curr. Med. Chem. 7, 211-247 (2000).
non-Patent Document 7: Lorenz, W. W. et al. Proc. Natl. Acad. Sci. USA 88, 4438-4442 (1991).
non-Patent Document 8: Mathews, J. C. et al. Biochemistry 16, 85-91 (1977).
non-Patent Document 9: Paulmurugan, R. & Gambhir, S. S. Anal. Chem. 75, 1584-1589 (2003).
non-Patent Document 10: Kaihara, A. et al. Anal. Chem. 75, 4176-4181 (2003).
non-Patent Document 11: Bhaumik, S. & Gambhir, S. S. Proc. Natl. Acad. Sci. USA 99, 377-382 (2002).
non-Patent Document 12: Sun, L. et al. Appl. Environ. Microbiol. 67, 1025-1029 (2001).
non-Patent Document 13: Wu, H. et al. Proc. Natl. Acad. Sci. USA 95, 9226-9231 (1998).
non-Patent Document 14: Yang, J. et al. Proc. Natl. Acad. Sci. USA 100, 3513-3518 (2003).
non-Patent Document 15: Giriat, I. & Muir, T. W. J. Am. Chem. Soc. 125, 7180-7181 (2003).
non-Patent Document 16: Chin, H. G. et al. Proc. Natl. Acad. Sci. USA 100, 4510-4515 (2003).
non-Patent Document 17: Ozawa, T. et al. Anal. Chem. 73, 5866-5874 (2001).
non-Patent Document 18: Ozawa, T. et al. Anal. Chem. 73, 2516-2521 (2001).
non-Patent Document 19: Ozawa, T. et al. Nat. Biotechnol. 21, 287-293 (2003).
non-Patent Document 20: Paulmurugan, R. et al. Proc. Natl. Acad. Sci. USA 99, 15608-15613 (2002).
non-Patent Document 21: Massoud, T. F. & Gambhir, S. S. Genes Dev. 17, 545-580 (2003).
non-Patent Document 22: Greer, L. F., 3rd & Szalay, A. A. Luminescence 17, 43-74 (2002).
non-Patent Document 23: Weissleder, R. & Ntziachrostos, V. Nat. Med. 9, 123-128 (2003).

### Disclosure of the Invention

Under the foregoing circumstances, the invention of this application has been made, and it aims to provide, upon solving the problems associated with the ordinary techniques, a convenient and highly accurate method for detecting protein nuclear transport induced by endogenous and exogenous substances in local areas of living cells or living non-human animals.

This application first provides, for solving the foregoing problems, a pair of probes for detecting and quantifying protein nuclear transport induced by action of a bioactive substance, comprising
Probe I in which a protein whose nuclear transport is to be detected or quantified is connected to an N-terminal end or a C-terminal end of a fusion protein [intein-C/reporter protein-C] wherein at least a C-terminal side polypeptide of an intein and a C-terminal side polypeptide of a reporter protein are connected in this order, and
Probe II in which a nuclear localization signal is connected to an N-terminal end or a C-terminal end of a fusion protein [reporter protein-N/intein-N] wherein at least the remaining N-terminal side polypeptide of the reporter protein and the remaining N-terminal side polypeptide of the intein are connected in this order. This application second provides a pair of probes for detecting and quantifying protein nuclear transport induced by action of a bioactive substance, comprising
   Probe I in which a protein whose nuclear transport is to be detected or quantified is connected to an N-terminal end or a C-terminal end of a fusion protein [reporter protein-N/intein-N] wherein at least a N-terminal side polypeptide of a reporter protein and a N-terminal side polypeptide of an intein are connected in this order, and
   Probe II in which a nuclear localization signal is connected to an N-terminal end or a C-terminal end of a fusion protein [intein-C/reporter protein-C] wherein at least the remaining C-terminal side polypeptide of the intein and the remaining C-terminal side polypeptide of the reporter protein are connected in this order.

This application third provides the pair of probes, wherein the intein is a DnaE intein derived from blue-green algae, and fourth provides the pair of probes, wherein the reporter protein is luciferase.

The invention is defined in the claims.

The invention of this application provides a method for detecting and quantifying protein nuclear transport induced by action of a bioactive substance, which comprises
making Probe I of any of foregoing pair of probes and the bioactive substance coexist in the cytosol,
localizing Probe II in the nucleus, and
measuring a signal of the reporter protein within the nucleus.

The invention of this application provides the method for detecting and quantifying protein nuclear transport, wherein polynucleotides expressing any of foregoing pair of probes are introduced into a cell thereby making Probe I and the bioactive substance coexist in the cytosol and localizing Probe II in the nucleus, and provides the method for detecting and quantifying protein nuclear transport, wherein polynucleotides expressing any of the foregoing pairs of probes are introduced into a non-human animal multipotent cell and the cell is subjected to ontogenesis thereby making Probe I and the bioactive substance coexist in the cytosol and localizing Probe II in the nucleus in all cells of the animal or its progeny.

The invention of this application provides a method for screening a protein nuclear transport-inducing substance, which comprises
introducing Probe I of any of the foregoing pair of probes into the cytosol,
localizing Probe II in the nucleus,
introducing a nuclear transport-inducing candidate substance into the cytosol, and
measuring a signal of the reporter protein in the nucleus.

The invention of this application provides a method for screening a protein nuclear transport-inhibiting substance, which comprises:
introducing Probe I of any of the foregoing pair of probes into the cytosol,
localizing Probe II in the nucleus,
introducing a nuclear transport-inhibiting candidate substance into the cytosol,
further introducing a nuclear transport-inducing substance into the cytosol,
measuring a signal of the reporter protein in the nucleus, and
comparing the signal with a signal of the reporter protein obtained by introducing only the protein nuclear transport-inducing substance into the cytosol.

The invention of this application provides the screening method, wherein polynucleotides expressing any of the foregoing pair of probes are introduced into the cell thereby introducing Probe I into the cytosol and localizing Probe II in the nucleus, and provides the screening method, wherein polynucleotides expressing any of the foregoing pair of probes are introduced into a non-human animal multipotent cell and the cell is subjected to ontogenesis thereby introducing Probe I in the cytosol and localizing Probe II in the nucleus in all cells of the animal or its progeny.

### Brief Description of the Drawings

Fig. 1 is a schematic view showing a structure and an action of the pair of probes for detecting protein nuclear transport in this invention.
Fig. 2 is a fluorescence microscope image and a transmission microscope image of COS-7 cells transiently transfected with pcDRc-AR expression vector. (a: anti-AR antibody; b: anti-Flag antibody; 1: without addition of DHT; 2: addition of DHT)
Fig. 3 is Western blot in Examples of this invention. (a: protein extract of COS-7 cells; b: cells with probes I and II co-expressed (addition of DHT); c: cells with probes I and II co-expressed (without addition of DHT))
Fig. 4 is dose-response curves of DHT added to cells based on the luminescence intensities of reconstituted RLuc.
Fig. 5 is dose-response curves of various endogenous hormones and synthetic chemicals based on the luminescence intensities of reconstituted RLuc. (a: endogenous hormones; b: synthetic chemicals; c: inhibitors of protein nuclear transport)
Fig. 6 is a CCD image showing protein nuclear transport in mouse carrying transiently transfected COS-7 cells. (1: no probe; 2: only Probe II is expressed; 3: only Probe I is expressed; 4: probes I and II are expressed)
Fig. 7 is the average of photon counts from mouse carrying transiently transfected COS-7 cells. (1: no probe; 2: only Probe II is expressed; 3: only Probe I is expressed; 4: probes I and II are expressed)
Fig. 8 is a time course of luminescence change after i.p. injection of coelenterazine in mice carrying transiently transfected COS-7 cells. (1: no probe; 2: only Probe II is expressed; 3: only Probe I is expressed; 4: probes I and II are expressed)
Fig. 9 is a CCD image of mice carrying COS-7 cells cotransfected with probes I and II in the presence or absence of DHT.
Fig. 10 is photon counts from mice carrying COS-7 cells cotransfected with probes I and II in the presence or absence of DHT. (average of four mice)
Fig. 11 is a CCD image showing inhibition of protein nuclear transport with procymidone and PCB in mice carrying COS-7 cells cotransfected with probes I and II. (a: control (DMSO stimulus); b: DHT; C: DHT+ procymidone; d: DHT+PCB)
Fig. 12 is a graph showing inhibition of protein nuclear transport with procymidone and PCB in mice carrying COS-7 cells cotransfected with probes I and II. (a: control (DMSO stimulus); b: DHT; C: DHT+ procymidone; d: DHT+PCB)
Fig. 13 is a schematic view showing a structure and an action of the pair of probes for detecting protein nuclear transport in this invention (glucocorticoid receptor as a protein).
Fig. 14 is a fluorescence microscope image and transmission microscope image of NIH3T3 cells transiently transfected with pcDRc-GR expression vector (a: anti-GR antibody; b: anti-Flag antibody; Corti-: no addition of corticosterone; Corti+: addition of corticosterone)
Fig. 15 is dose-response curves of corticosterone, dexamethasone, cortisol, progesterone, testosterone, 2DG, DHT, Mf(RU486), E2 and C-terminal only (corti) added to cells based on the fluorescence intensities of reconstituted RLuc.
Fig. 16 is a CCD image showing a protein nuclear transport in mouse carrying transiently transfected NIH3T3 cells. (1: no probe; 2: only Probe II is expressed; only Probe I is expressed; 4: probes I and II are expressed)
Fig. 17 is a schematic view showing a structure and an action of the pair of probes for detecting a protein nuclear transport in this invention (Sterol Regulatory Element-Binding Protein-2 as a protein).
Fig. 18 is a microscope image and a transmission microscope image of cells transiently transfected with pcDRc-SREBP expression vector. (a: detection of N-terminal protein; b: detection of SREBP-2; arrow 1: condition before translocation into the nucleus (localization); arrows 2 to 4: conditions after translocation into the nucleus; +chol: in the presence of cholesterol; -chol: in the absence of cholesterol)
Fig. 19 is dose-response curves of cholesterol added to cells based on the luminescence intensities of reconstituted RLuc.
Fig. 20 is a schematic view showing a structure and an action of the pair of probes for detecting protein nuclear transport in this invention (Signal Transducer and Activator of Transcription 3 as a protein).
Fig. 21 is a fluorescence microscope image and a transmission microscope image of HEK293 cells transiently transfected with pcDRc-STAT-3 expression vector. (a: anti-Flag antibody; b: anti-STAT antibody; OSM-: no addition of OSM; OSM+: addition of OSM)

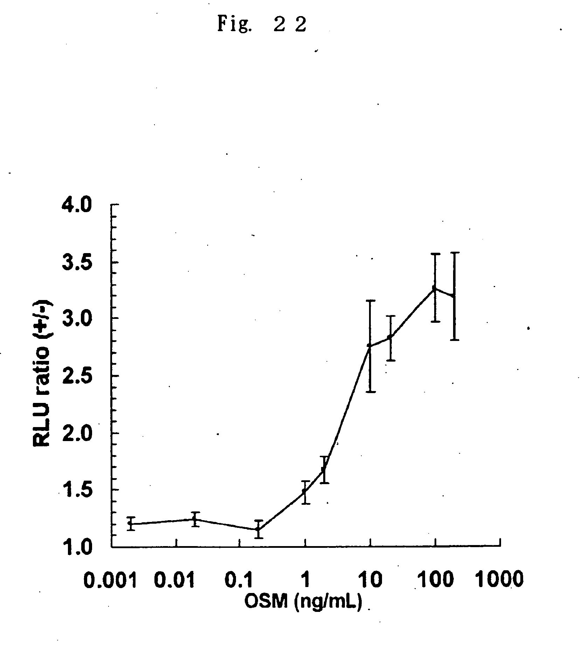

Fig. 22 is dose-response curves of OSM added to cells based on the fluorescence intensities of reconstituted RLuc.

Incidentally, the reference numerals in the drawings indicate the followings.
- 1:: pair of probes for detecting protein nuclear transport
11: Probe I
12: Probe II
- 2:: protein
- 3:: reporter protein
3c: C-terminal side polypeptide of reporter protein
3n: N-terminal side polypeptide of reporter protein
- 4:: intein
4c: C-terminal side polypeptide of intein
4n: N-terminal side polypeptide of intein
- 5:: nuclear localization signal
- 6:: bioactive substance, protein nuclear transport-inducing substance
6': protein nuclear transport-inducing candidate substance
6": protein nuclear transport-inhibiting candidate substance
- 7:: linker
- 8:: nuclear membrane

### Best Mode for Carrying Out the Invention

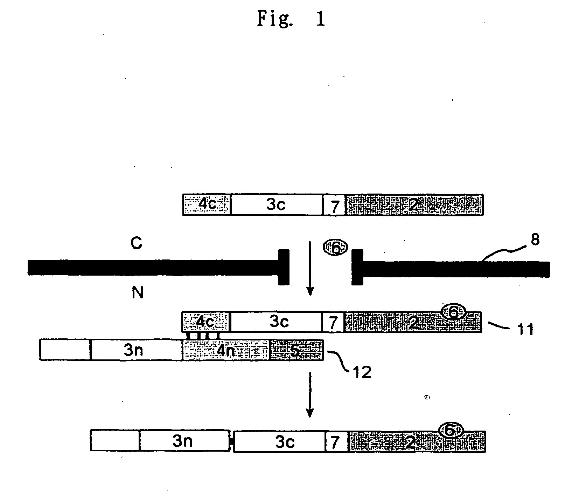

Fig. 1 has shown a schematic view indicating a principle of the pair of probes for detecting protein nuclear transport in this invention. That is, the pair of probes *1* comprises Probe I *(11)* and Probe II *(12).* In Probe I *(11),* target protein *2* is connected to an N-terminal end or a C-terminal end of a fusion protein [intein-C/reporter protein-C] in which at least a C-terminal side polypeptide *4c* of intein *4* and a C-terminal side polypeptide *3c* of reporter protein *3* are connected in this order. In Probe II *(12)*, nuclear localization signal *5* is connected to an N-terminal end or a C-terminal end of fusion protein [reporter protein-N/intein-N] in which at least the remaining N-terminal side polypeptide 3*n* of reporter protein 3 and the remaining N-terminal side polypeptide *4n* of intein *4* are connected in this order. Alternatively, in Probe I *(11)*, target protein *2* is connected to an N-terminal end or a C-terminal end of fusion protein [reporter protein-N/intein-N] in which at least an N-terminal side polypeptide *3n* of reporter protein *3* and an N-terminal side polypeptide *4n* of intein 4 are connected in this order, and in Probe II *(12),* nuclear localization signal *5* is connected to an N-terminal end or a C-terminal end of fusion protein [intein-C/reporter protein-C] in which at least the remaining C-terminal side polypeptide *4c* of intein *4* and the remaining C-terminal side polypeptide *3c* of reporter protein *3* are connected in this order.

When introduced the pair of probes into the cell, Probe I *(11)* is localized in the cytosol, and Probe II *(12)* in the nucleus. When the protein 2 in Probe I *(11)* is bound to nuclear transport-inducing substance *6,* Probe I *(11)* translocates from cytosol *C* into nucleus *N* and approaches Probe II *(12).* Then, two sites *4c, 4n* of intein *4* approach and are correctly folded, whereby splicing takes place, intein *4* is cut out from Probe I *(11)* and Probe II *(12)*, and two sites *3c*, *3n* of reporter protein *3* are connected via a peptide linkage to reconstitute reporter protein *3*.

Meanwhile, when protein *2* in Probe I *(11)* is not bound to nuclear transport-inducing substance 6, the splicing of intein *4* does not take place, and reporter protein *3* is not reconstituted accordingly.

Therefore, the translocation of protein *2* into the nucleus can be detected by measuring the signal of reporter protein *3*.

In the foregoing pair of probes, Probe I (*11*) is a tandem fusion protein having a structure of [protein *2*/intein-C *4c*/reporter protein-C *3c*], [intein-C (4c)/reporter protein-C (3c)/protein (2)], [protein 2/reporter protein-N *3n*/intein-N *4n*] or [reporter protein-N 3*n*/intein-N 4*n*/protein *2*]. Such Probe I may contain, other than these elements, a polypeptide or the like as linker sequence 7 between the respective elements.

Likewise, Probe II *(12)* is a tandem fusion protein having a structure of [reporter protein 3*n*/intein-N 4*n*/nuclear localization signal *5*], [nuclear localization signal *5*/reporter protein-N *3n*/intein-N *4n*], [intein-C *4c*/reporter protein-C *3c*/nuclear localization signal *5*] or [nuclear localization signal *5*/intein-C *4c*/reporter protein-C *3c*]. Probe II may contain, other than these elements, a polypeptide or the like as a linker sequence *7* between the respective elements.

In the pair of probes *1,* protein *2* contained in Probe I (*11*) is not particularly limited so long as its translocation into the nucleus is to be detected and quantified. For example, androgen receptor (AR), a well-known nuclear hormone receptor is employable. AR is known to translocate from the cytosol into the nucleus by being bound to 5α-dihydrotestosterone (DHT) (non-Patent Document 6). Further, allyl hydrocarbon receptor (AhR) known to translocate into the nucleus by a dioxin, thyroid hormone receptor (ThR) known to translocate into the nucleus by thyroid hormone, sterol responsive element binding protein (SREBP) known as a cholesterol sensor, mitogen-activated protein kinase (MAPK) involved in a cell growth signal, nuclear factor-κB (NFκB) inducing apoptosis and the like are employable.

In the pair of probes *1*, nuclear localization signal *5* contained in Probe II *(12)* is for localizing Probe II *(12)* in the nucleus *N* when Probe II *(12)* is introduced into cells, and its structure and sequence are not particularly limited. Specifically, SEQ ID NO: 1 is employable. Of course, other than this, known nuclear localization sequences (NLS) such as nucleoplasmin-derived sequence (SEQ ID NO: 2) and HIV-1 Rev-derived sequence (SEQ ID NO: 3) can be employed.

In the pair of probes *1*, reporter protein *3*, which is divided into C-terminal side polypeptide *3c* and N-terminal side polypeptide *3n* for Probe I *(11)* and Probe II *(12),* may be any reporter protein so long as peptide binding directly takes place by splicing with intein *4* to allow reconstitution of split reporter protein, thereby enabling analysis. For example, a fluorescent protein or a luminous catalytic enzyme is preferably employed. A photoprotein such as a green fluorescent protein (GFP) and its derivatives is preferable because it emits light and allows visual analysis. The luminous catalytic enzyme such as luciferase is also preferable because it forms an active center by being connected and emits light easily detectable with a luminometer. Renilla luciferase (RLuc) is especially preferable because its molecular weight is as low as 36-kDa and ATP or posttranslational modification is not necessary for its activity (non-Patent Documents 7 and 8). In order that the N- and C-terminals of split RLuc respectively do not exhibit fluorescence alone but recover its activity by being bound, it is advisable to split at the RLuc active center. Specifically, it is reported that the active center is preferably dissected between G229 and K230 (non-Patent Documents 9 and 10). Further, Rluc takes an advantage of its substrate, jejunal luciferin (coelenterazine), which rapidly penetrates through cell membranes and produces luminescence intensity sufficient for in vivo visualization (non-Patent Document 11).

Moreover, in the pair of probes *1*, known inteins derived from various organisms can be employed as C-terminal side polypeptide *4c* and N-terminal side polypeptide 4n for Probes I *(11)* and Probe II *(12)*. Intein 4 is preferably a site-specific endonuclease so as to be automatically cut out when protein *2* of Probe I *(11)* interacts with nuclear transport-inducing substance *6* and Probe I *(11)* translocates into the nucleus (N) to approach Probe II *(12)*. Specifically, yeast VMA-derived intein and blue-green argae-derived DnaE intein are preferable. Of these, blue-green argae-derived DnaE is easy to handle because the DNA sequence of PCC6803 strain is known, as well as it is natural split intein (non-Patent Documents 12 to 20).

In the pair of probes *1,* for the splicing of intein *4* to effectively occur, it is required that when Probe I *(11)* and Probe II *(12)* approach in the nucleus, the two sites involved in the protein splicing are correctly folded and accurately arranged. Therefore, while any intein derived from organisms may directly be used, it may be preferable to design the intein for easy splicing by converting or deleting some amino acid residues or introducing an appropriate linker sequence.

In the pair of probes *1*, protein *2* is connected to the N-terminal end or the C-terminal end of fusion protein [intein-C *4c*/reporter protein-C *3c*] in which a split polypeptide fragment of intein *4* (for example, C-terminal side: *4c*) is connected to a split polypeptide fragment of reporter protein *3* (in this case, *3c*) to construct Probe I *(11)*.

Nuclear localization signal *5* is connected to the N-terminal end or the C-terminal end of fusion protein [reporter protein-N *3n*/intein-N *4n*] in which another split polypeptide fragment of reporter protein *3* (*3n* in the foregoing example) is connected to another split polypeptide fragment of intein *4* (in this case, N-terminal side: *4n*) in this order to construct the Probe II *(12).*

In such a pair of probes *1*, connecting manner of intein-C *4c* and reporter protein-C *3c;* fusion protein [intein-C (4c)/reporter protein-C (3c)] and protein (2); reporter protein-N *3n* and intein-N *4n*; the fusion protein [reporter protein-N 3n/intein-N *4n*] and nuclear localization signal 5 may be arbitrary unless influencing protein *2*, nuclear localization signal *5*, Probes *(11)*, *(12)* and the like. For example, chemical, biochemical or gene engineering method can be applied.

In the invention of this application, a method for detecting and quantifying the translocation of protein *2* into the nucleus using pair of probes *1* is provided. Specifically, Probe I *(11)* in the pair of probes *1* is caused to coexist with bioactive substance *6* in the cytosol, and Probe II *(12)* is localized in the nucleus (N) thereby measuring the signal of reporter protein *3* in the nucleus (N).

In this method for detecting and quantifying protein nuclear transport, Probe I *(11)* is retained in the cytosol (C), and Probe II *(12)* is localized in the nucleus (N) by nuclear localization signal *5.* Protein *2* recognizes and binds to bioactive substance *6*, whereby Probe I *(11)* translocates from the cytosol (C) into the nucleus. Then, Probe I *(11)* and Probe II *(12)* approach, and intein *4* therein is cut out by splicing to reconstitute reporter protein *3*. Accordingly, when the signal of reporter protein *3* is measured, the translocation of protein *2* into the nucleus by bioactive substance *6* can be detected with high accuracy. A calibration curve of a relation between the concentrations and the signal intensities of reporter protein 3 allows quantifying the degree of the translocation of protein *2* into the nucleus.

In the method of this invention, for coexisting Probe I *(11)* and bioactive substance *6* in the cytosol (C) and localizing Probe II *(12)* in the nucleus (N), polynucleotides expressing the pair of probes *1* may be introduced into cells. Alternatively, it is also possible that the polynucleotides expressing the pair of probes *1* are introduced into non-human animal multipotent cells and the cells are subjected to ontogenesis, whereby in all cells of this animal or its progeny, Probe I *(11)* and bioactive substance *6* are caused to coexist and Probe II *(12)* is localized in the nucleus (N).

The invention of this application also provides a method for screening a substance that induces translocation of protein *2* into the nucleus. That is, Probe I *(11)* of the pairs of probes *1* is introduced into the cytosol (C), Probe II *(12)* is localized in the nucleus (N), and nuclear transport-inducing candidate substance *6'* is made to coexist in the cytosol (C). Subsequently, the signal of reporter protein *3* in the nucleus (N) is measured, whereby the nuclear transport-inducing substance can be screened.

When nuclear transport-inducing candidate substance *6'* interacts with protein *2* in Probe I *(11)* to induce the nuclear translocation of protein *2*, Probe I *(11)* translocates from the cytosol (C) into the nucleus (N) and approaches Probe II *(12)* localized in the nucleus (N). Consequently, a half polypeptide of intein *4c* and another half polypeptide of intein *4n* approach to cause the splicing, and divided reporter proteins *3c* and *3n* are peptide-bonded to reconstitute reporter protein *3*. Accordingly, by measuring the signal of reporter protein *3*, it can be confirmed whether or not the candidate substance is a nuclear transport -inducing substance.

For introducing Probe I *(11)* into the cytosol (C) and localizing Probe II *(12)* in the nucleus (N) in this screening method, a method for introducing the polynucleotides expressing pair of probes *1* into cell may be applied. Alternatively, by introducing the polynucleotides expressing pair of probes *1* into a non-human animal multipotent cell and the cell is subjected to ontogenesis, Probe I *(11)* is introduced into the cytosol and Probe II *(12)* is localized in the nucleus (N) in all cells of this animal or its progeny.

The invention of this application further provides a method for screening a protein nuclear transport-inhibiting substance. That is, Probe I *(11)* of pairs of probes *1* is introduced into the cytosol (C), Probe II *(12)* is localized in the nucleus (N), and nuclear transport-inhibiting candidate substance *6"* is first introduced into the cytosol (C). Protein nuclear transport-inducing substance *6* is then introduced into the cytosol and the signal of reporter protein *3* in the nucleus (N) is measured. The result thereof is compared with the signal of reporter protein *3* at introduction of protein nuclear transport-inducing substance *6* alone into the cytosol (C), whereby the nuclear transport-inhibiting substance can be screened.

When the nuclear transport-inhibiting candidate substance *6"* inhibits the binding of protein *2* in Probe I *(11)* and protein nuclear transport-inducing substance *6*, the signal intensity of reporter protein *3* is decreased in comparison to that at the absence of the candidate substance (namely at the presence of nuclear transport-inducing substance *6* only). Accordingly, the substance reducing the signal of reporter protein *3* can be estimated as the nuclear transport-inhibiting substance.

For introducing Probe I *(11)* into the cytosol (C) and localizing Probe II *(12)* in the nucleus (N) in this screening method too, a method for introducing the polynucleotides expressing pair of probes *1* into cell may be applied. Alternatively, by introducing the polynucleotides expressing pair of probes *1* into a non-human animal multipotent cell and the cell is subjected to ontogenesis, Probe I *(11)* is introduced into the cytosol and Probe II *(12)* is localized in the nucleus (N) in all cells of this animal or its progeny.

Embodiments of this invention are described in more detail below by referring to Examples along the attached drawings. Of course, this invention is not limited by the following Examples, and for the details, various embodiments are, needless to say, possible.

### Examples

### [Procedures]

### (1) Construction of Plasmids

The cDNA-encoding N-terminal domain of Rluc (Rluc-N; 1~229 aa) was modified by PCR to introduce the peptide (KFAEYC: SEQ ID NO: 4) to the C terminus of Rluc-N and to introduce the FLAG epitope (DYKDDDDK: SEQ ID NO: 5) to the N terminus of Rluc-N. The cDNA encoding the modified Rluc-N was fused to a cDNA encoding the N-terminal splicing domain of DnaE (DnaE-N; 1~123 aa) with a native *Hind*III site. The cDNA of C terminus of DnaE-N was fused to a cDNA of a nuclear localization signal [NLS; (DPKKKRKV)₃: SEQ ID NO: 1]with the *Nco*I site.

The cDNA-encoding C-terminal domain of Rluc (Rluc-C; 230~311 aa) was modified by PCR to introduce the peptide, FNLSH (SEQ ID NO: 6), and the unique enzyme site, MunI, to the N terminus of Rluc-C and to introduce a linker (GGGGSG: SEQ ID NO: 7) and a unique enzyme site, NotI, to the C terminus of Rluc-C.

The cDNA encoding AR (1~918 aa) was modified by PCR to add a unique enzyme site, NotI, at its N-terminal end and XhoI at its C-terminal end. The cDNA encoding the modified Rluc-C was fused to a cDNA encoding the C-terminal splicing domain of DnaE (DnaE-C; 1~36 aa) with the MunI site and to cDNA encoding the full AR with NotI.

The cDNAs were subcloned into the expression vector pcDNA 3.1(+) (Invitrogen) at the unique enzyme sites *Bam*HI and *Xho*I.

The PCR products were sequenced to ensure fidelity with a BigDye Terminator Cycle Sequencing kit and a genetic analyzer ABI Prism310 (PE Biosystems).

### (2) Cell Culture and Transfection

COS-7 cells were cultured in DMEM supplemented with 10% steroid-free FBS (a charcoal-extracted FBS) and 1% penicillin/streptomycin at 37°C in 5% CO₂.

The cells seeded in 12-well culture plates were transfected with 2 µg of constructed plasmids using a lipofection reagent, lipofectAMINE2000 (Invitrogen).

### (3) Western Blot Analysis

COS-7 cells were transfected with either pcRDn-NLS or pcDRc-AR and incubated for 24 h. The cells were washed once in PBS and lysed in 200 µl of lysis buffer (1% SDS/10% glycerol/10% 2-mercaptoethanol/0.001% bromophenol blue/50 mM Tris-HCl, pH 6.8). Equal amounts of the samples were electrophoresed in 6% acrylamide gels, transferred to nitrocellulose membrane, and blotted with mouse anti-AR antibody (Santa Cruz Biotechnology). The blots were incubated with alkaline phosphatase-conjugated secondary antibodies and visualized by chemiluminescence (New England Biolabs).

### (4) Immunocytochemistry

COS-7 cells were cultured on microscope glass slides (2 x 10⁵ cells per slide) and were transfected with the constructed plasmids. The transfected cells were fixed with a 3% paraformaldehyde solution. The cells were blocked with 0.2% fish skin gelatin and then incubated with mouse anti-AR antibody (Santa Cruz Biotechnology) or mouse anti-FLAG antibody (Sigma). The antibodies were reacted with Cy-5-conjugated secondary antibody, and then recorded by using a confocal laser-scanning microscope (LSM510; Zeiss) fitted with 647 nm cut-off filter and an 665 nm I.P. filter.

### (5) Cell-Based in Vitro Assay

COS-7 cells were cotransfected with the plasmids and incubated for 12 h. The medium was replaced with DMEM supplemented with 10% FBS, and 24 h after the replacement, steroid hormones or synthetic chemicals were added to each well.

After the COS-7 cells were extensively incubated for 2 hours, the cells were harvested, and luciferase activity was evaluated by using the *Renilla* luciferase assay kit (Promega) with a luminometer (Minilumat LB9506; Berthold, GmbH) with an integration time of 20 sec.

### (6) In Vivo Imaging of Living Mice

The COS-7 cells were transfected with the constructed plasmid pcRDn-NLS and pcDRc-AR, respectively, or cotransfected with pcRDn-NLS and pcDRc-AR. The cells were harvested after incubation in the DMEM with 10% FBS for 12 h after transfection. The cells were suspended in DMEM, and an aliquot of 1 x 10⁶ cells was implanted in four different sites on the back of anesthetized BALB/c nude mice (female, 5 weeks old, about 17 g body weight).

Twelve hours after cell implantation, 100 µl of DHT (100 µg/kg of body weight) or 1.0% (vol/vil) DMSO (vehicle) was injected i.p. Two hours after injection, 100 µl of coelenterazine (2.8 mg/kg of body weight) was injected i.p., and the mice were imaged at 2-min intervals.

For examining DHT dependence on the Rluc activity, two groups of nude mice were injected with the COS-7 cells (1 x 10⁶ cells) cotransfected with plasmids pcRDn-NLS and pcDRc-AR directly into the backs of the mice. Of the two groups, the first mouse group was injected i.p. with 100 µl of 1.0% (vol/vol) DMSO (vehicle). The second group was injected with 100 µl of DHT (100 µg/kg of body weight). After 3 h, 100 µl of coelenterazine (1.4 mg/kg of body weight) was injected i.p., and the mice were imaged 10 min later (n = 4).

For testing the brains of mice, COS-7 cells (1 x 10⁵ cells) cotransfected with plasmids pcRDn-NLS and pcDRc-AR were implanted in the forebrain of the nude mice at a depth of 3 mm through a 1-mm burrhole. Soon after implantation, the first and second mouse groups were injected i.p. with 100 µl of 1.0% DMSO. The third and fourth mouse groups were injected i.p. with 100 µl of procymidone and polychlorinated biphenyls (PCB) (10 mg/kg of body weight) dissolved in 1.0% DMSO, respectively.

One hour after injection, the second and third groups of mice were injected i.p. with 100 µl of DHT (10 µg/kg of body weight) in 1.0% DMSO. Two hours after injection, 10 µl of coelenterazine (0.14 mg/kg of body weight) was injected intracerebrally in the mice, and representatives from each mouse group (n = 3) were imaged in 2-min intervals.

All mice were imaged by using a cooled CCD camera (IVIS 100 system, Xenogen). Photons emitted from the cells implanted in the mice were collected and integrated for a period of 1 min. Images were obtained by using LIVING IMAGE software (Xenogen). To quantify the measured light, regions of interest were drawn over the cell-implanted area, and the mean luminescence intensities (photons per sec per cm²) were evaluated.

### <Example 1>

To ensure that AR connected to the C-terminal domains of RLuc and DnaE (namely, Probe I) is correctly localized in intracellular organelles within animal cells, the COS-7 cells were transiently transfected with pcDRc-AR expression vector.

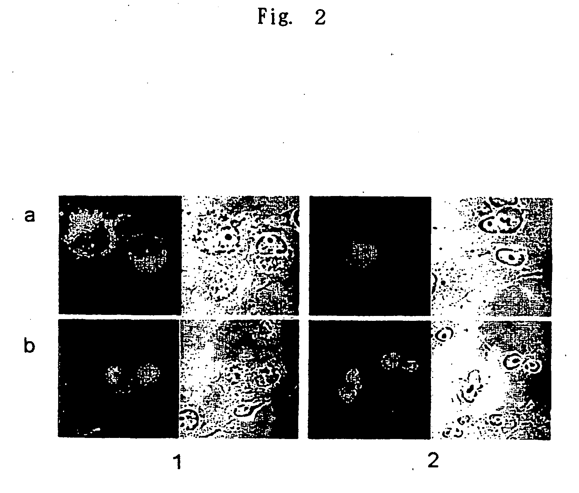

In the absence of DHT, Probe I was predominantly in cytoplasmic (Fig. 2a-1), whereas addition of DHT resulted in the nuclear localization of Probe I (Fig. 2a-2).

In contrast, Probe II, the N-terminal domains Rluc and DnaE with nuclear localization signal (NLS), was permanently localized in the nucleus both in the presence and absence of DHT.

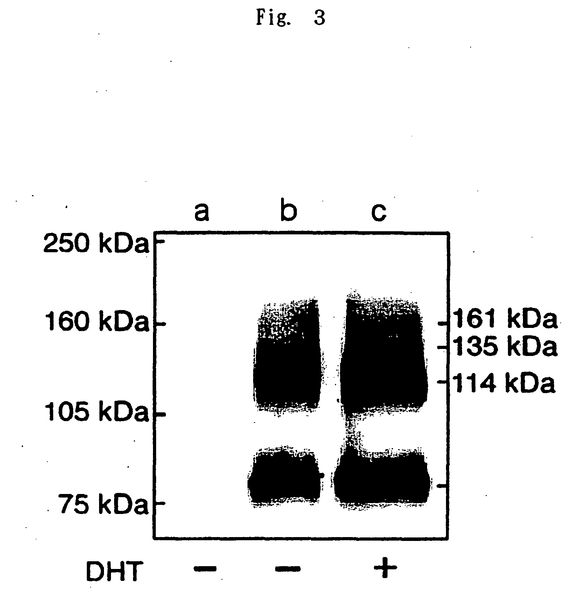

These observations were consistent with the results of Western blot analysis (Fig. 3). In crude extracts of the cells containing Probe I plasmid and Probe II plasmid in the absence of DHT, the AR antibody recognized only a specific component of an unspliced precursor, 115 kDa of AR tagged with Rluc-C and DnaE-C.

In the presence of DHT, however, the AR antibody recognized an unspliced precursor and 139 kDa and 160 kDa of a polypeptides, of which electrophoretic mobility was consistent with the predicted size of the products after protein splicing and splicing intermediate.

Given all these results, it was concluded that protein splicing occurred and the Rluc was reconstituted when the DHT-bound AR was translocated into the nucleus.

### <Example 2>

Next, to show that the pair of probes of this invention works for quantitative analysis of the extent of AR translocation into the nucleus, we tested the DHT-induced translocation of Probe I with the *in vitro* cell-based assay.

The COS-7 cells were transfected with both pcRDn-NLS and pcDRc-AR or only with pcDRc-AR, and differing concentrations of DHT were added to each microtiter well. The cells were harvested, and their lysates were mixed with a coelenterazine solution.

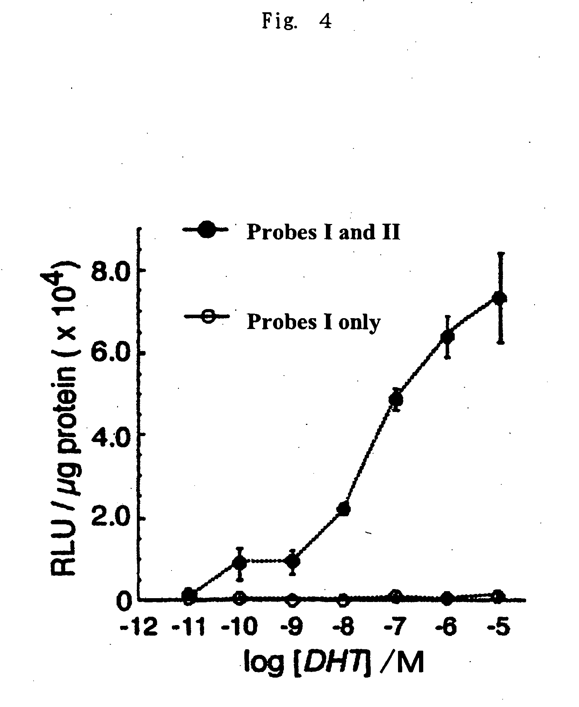

The luminescence signals increased with increasing the concentration of DHT and were strong enough to discriminate them from background luminescence (Fig. 4).

The results indicate that the pair of probes can be used for quantitative analysis of the extent of AR translocation into the nucleus.

### <Example 3>

The AR translocation into the nucleus with several endogenous hormones and synthetic chemicals was confirmed using the pair of probes of this invention.

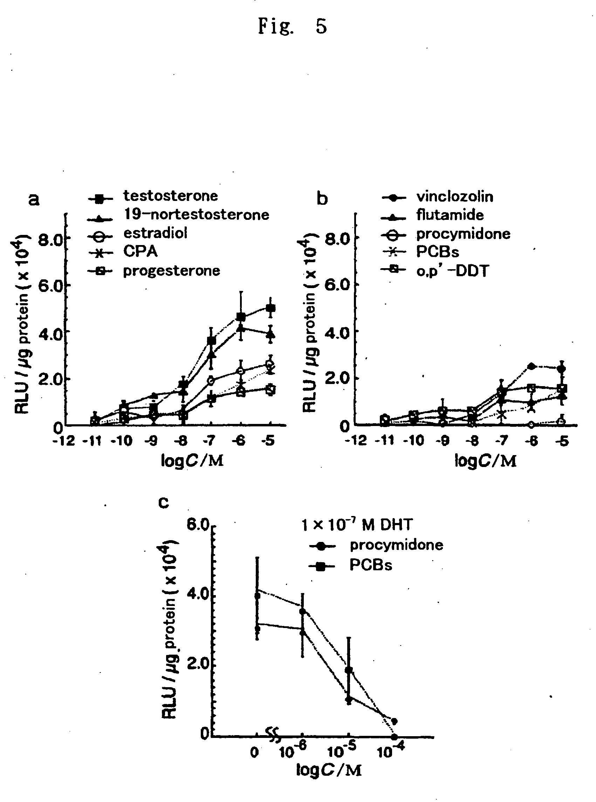

As shown in Fig. 5, endogenous androgens, such as testosterone and 19-nortestosterone, were found to induce high luminescence intensities over their concentration range from 10⁻⁸ to 10⁻⁵ M. The relative intensities with testosterone and 19-nortestosterone with their 10⁻⁶ M concentration were 72% and 64%, respectively, of those with the same concentration of DHT (Fig. 5a). Other endogenous steroid hormones, 17β-estradiol and progesterone, a synthetic steroid hormone; cyproterone acetate (CPA); and anti-androgens, vinclozolin and flutamide, also gave slight increases in the observed luminescence intensities (Fig. 5b). In contrast, procymidone did not induce any increase in its luminescence intensity over the concentration range tested.

Procymidone was also found to inhibit the DHT-induced strong luminescence. This indicates that procymidone did not trigger the nuclear import of AR even though it was bound to AR.

Of other tested chemicals, a PCB congener (Aroclor 1254) was found to hinder nuclear translocation of AR induced with DHT, whereas o,p'-DDT directed a small quantity of AR to the nucleus.

### <Example 4>

Next, it was confirmed that the distribution of chemical compounds in the organs of living animal could be observed using the pair of probes of this invention.

An optical bioluminescence imaging technique using a cooled CCD camera has advantages such as easiness of operation, short acquisition time and simultaneous measurement of plural mice, enabling high-speed imaging (non-Patent Documents 3 and 21 to 23).

To ensure that luminescence intensities provided by RLuc reconstituted in living mice are strong enough to detect with a CCD camera, 1,000,000 untransfected COS-7 cells (Reference Example), cells transiently transfected with pcRDn-NLS or pcDRc-AR or cells cotransfected with both pcRDn-NLS and pcDRc-AR were implanted into the coria on each site of the backs of living mice.

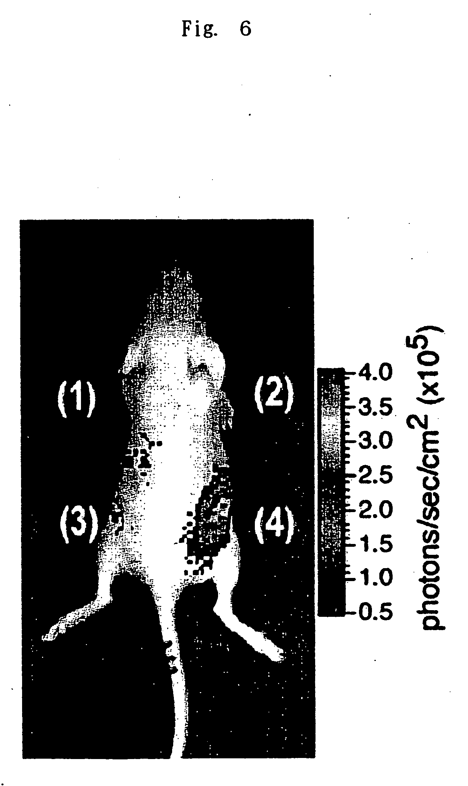

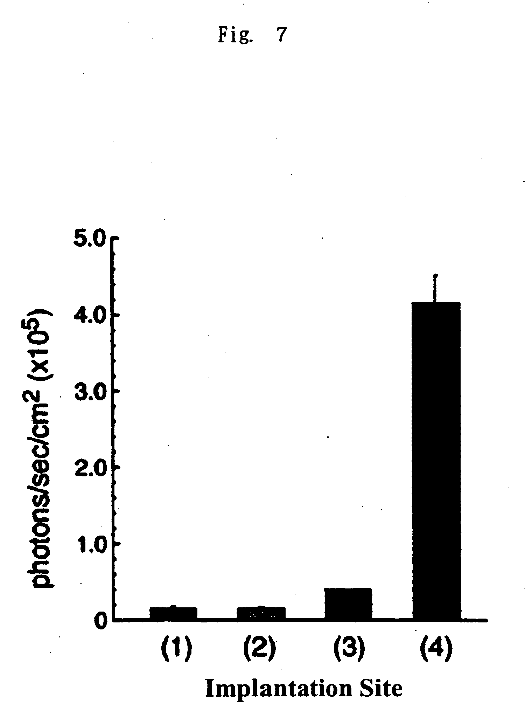

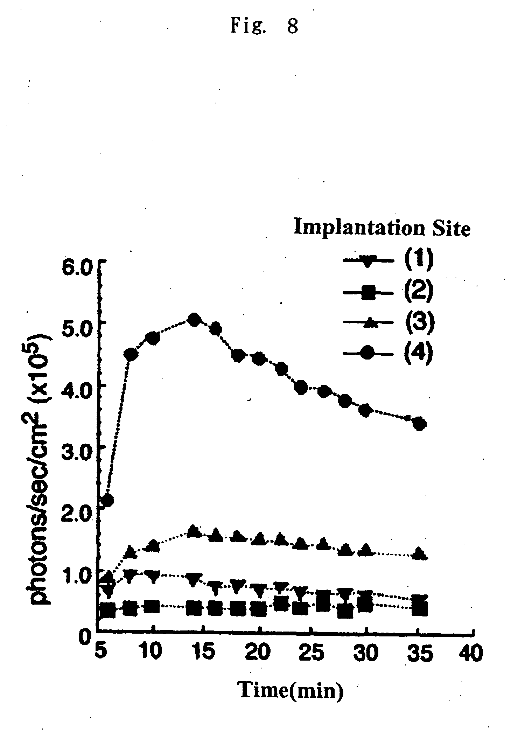

After 8-35 minutes, an observed cooled CCD image of mice showed a significant increase in the luminescence signal only from the site implanted with the cells cotransfected with pcRDn-NLS and pcDRc-AR (Figs. 6-8). The luminescence intensity obtained from the cotransfected cells reached the maximum at 15 min, which was 20 times higher than the background luminescence intensity from the cells transfected with either pcRDn-NLS or pcDRc-AR alone. The results demonstrate that high levels of bioluminescence can be detected from the implanted cells in living mice upon reconstitution of split Rluc.

### <Example 5>

To show the DHT-dependent nuclear import of AR in living mice, COS-7 cells transiently cotransfected with pcRDn-NLS and pcDRc-ARwere implanted on the back of mice.

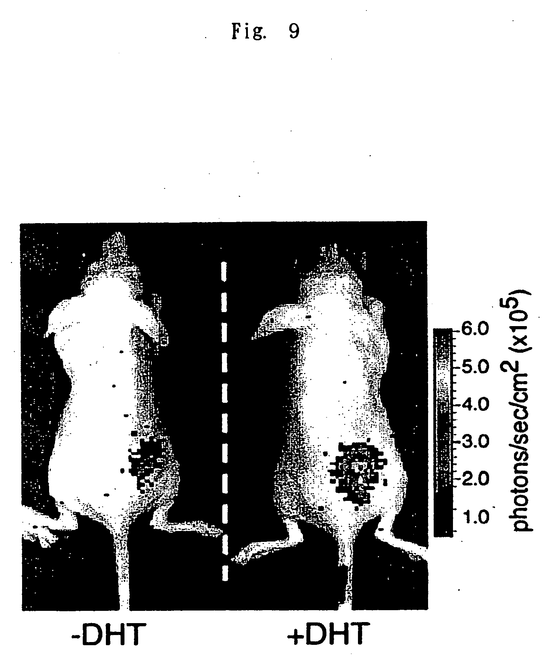

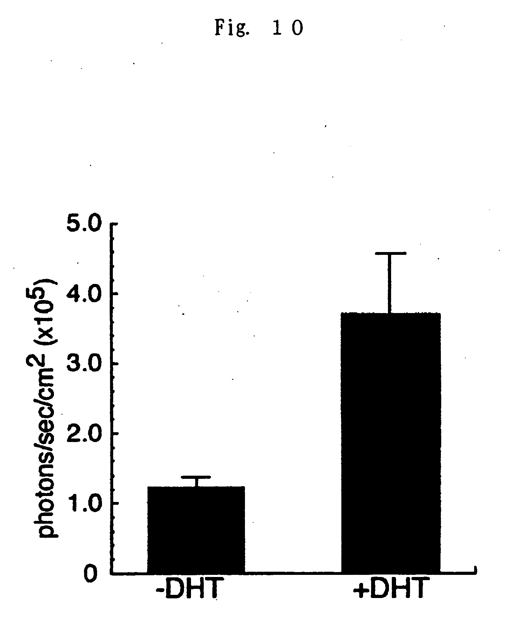

One group of mice (n=4) were stimulated with 1.0% DMSO (vehicle) for 2 hours, whereas the other group of mice (n=4) were stimulated with DHT (100 µg/kg of body weight) for the same period. Luminescence intensities obtained from the mice with DHT stimulation were 3.5 times higher than that with vehicle stimulation (Figs. 9 and 10).

The results demonstrate that it is possible to image and quantitatively evaluate the difference in the extent of AR translocation in living mice in the presence of DHT relative to its absence.

### <Example 6>

Further, the effects of inhibitors on AR translocation into the nucleus in the mouse brain were examined using the pair of probes of this invention.

The cells cotransfected with pcRDn-NLS and pcDRc-AR were implanted in the mouse brain at a depth of 3 mm, and i.p. injection was performed with 10⁻⁷ M DHT alone or different hormone mixtures of further adding procymidone or PCB at various concentrations to 1.0% DMSO.

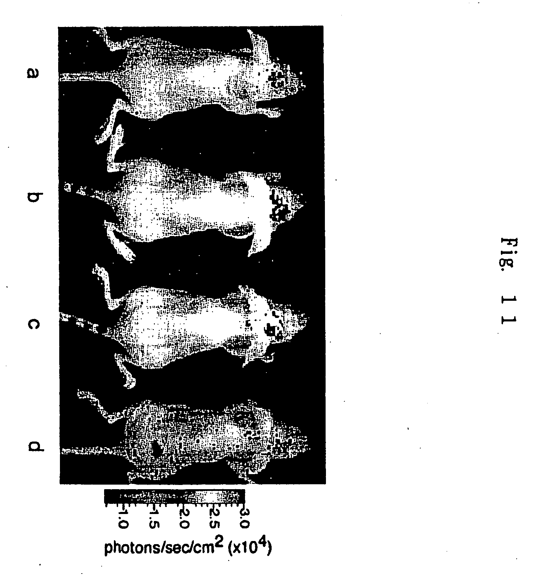

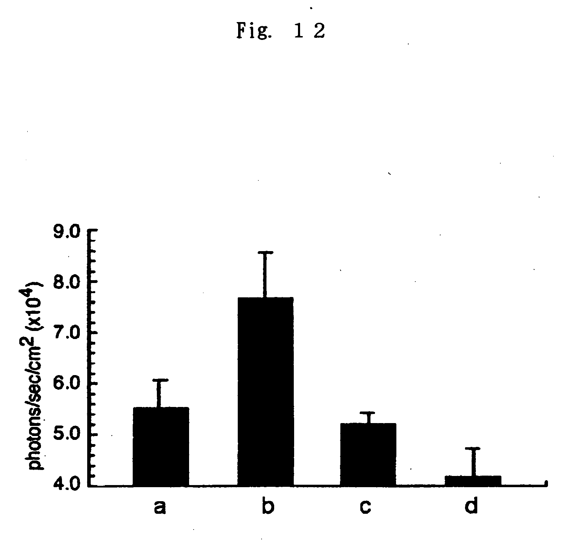

Induction of AR nuclear import by DHT resulted in a significant increase in photon count from the brains as compared to that with vehicle (Figs 11, 12).

Average from three mice indicates that the amount of the increase in bioluminescence was large in comparison to the luminescence intensity of the control. Stimulation with DHT together with procymidone and PCB, respectively, resulted in a decrease in luminescence intensity, demonstrating that procymidone and PCB had an ability to pass through the blood-brain barrier within 2 hours and hindered nuclear import of AR.

### <Example 7>

Translocation of glucocorticoid receptor (GR) into the nucleus was examined using a pair of probes of this invention.

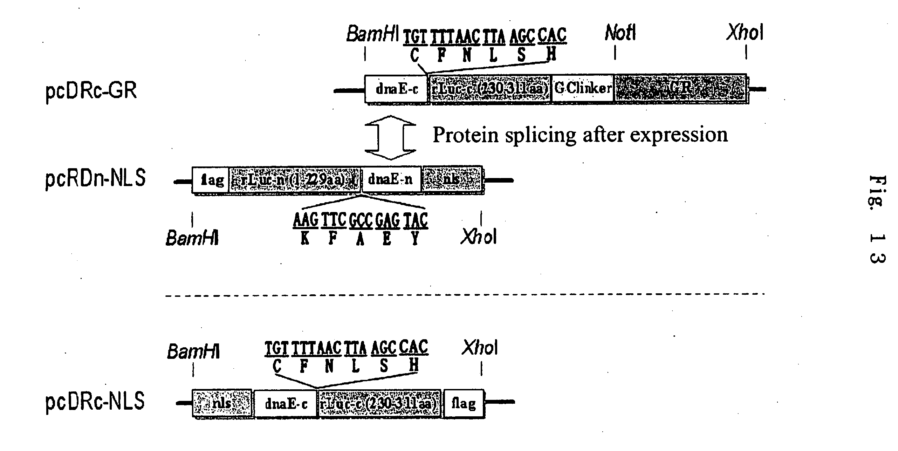

Plasmid pcDRc-AR in Examples 1 to 6 was cleaved with restriction endonucleases NotI and XhoI to remove the cDNA encoding AR, and a cDNA encoding glucocorticoid receptor (GR) was inserted at NotI and XhoI sites (Fig. 13). The same experiment as in case of AR was performed using the thus-constructed plasmid.

The cDNA-encoding N-terminal domain of Rluc (Rluc-N; 1~229 aa) was modified by PCR to introduce the peptide (KFAEY: SEQ ID NO: 8) to the C terminus of Rluc-N, and the cDNA-encoding C-terminal domain of Rluc (Rluc-C; 230~311 aa) was modified by PCR to introduce the peptide (CFNLSH: SEQ ID NO: 9) to the N terminus of Rluc-C.

### (1) Confirmation of GR localization

For confirming that GR connected to the C-terminal domains of RLuc and DnaE is correctly localized in intracellular organelles within animal cells, NIH3T3 cells were transfected with pcDRc-GR expression vector.

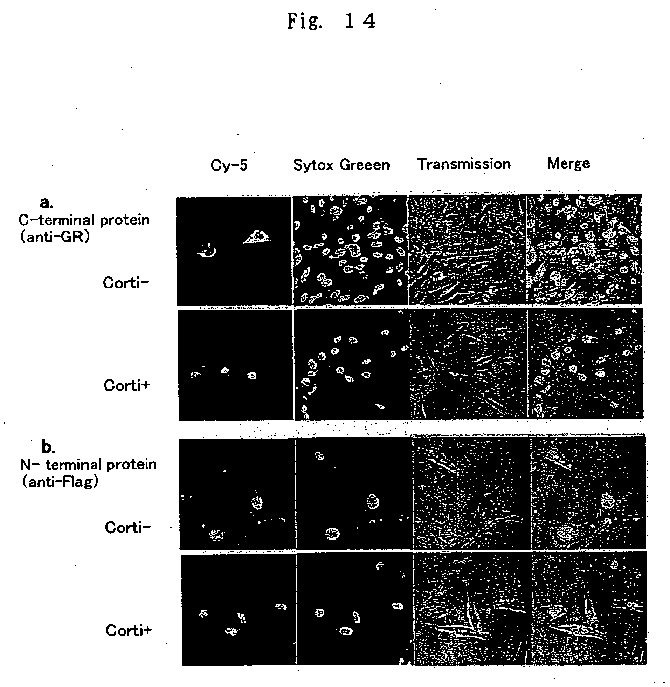

In the absence of corticosterone, Probe I was predominantly in cytoplasmic (Fig. 14, upper column, "Corti-"), whereas addition of corticosterone resulted in the nuclear localization of Probe I. (Fig. 14, second column, "Corti+").

### (2) Quantification of GR translocation into the nucleus

Next, to show that the pair of probes of this invention works for quantitative analysis of the extent of GR translocation into the nucleus, we tested the corticosterone -induced translocation of Probe I with the *in vitro* cell-based assay.

NIH3T3 cells were cotransfected with pcRDn-NLS and pcDRc-GR, and different concentrations of corticosterone were added to each microtiter well. The cells were harvested, and their lysates were mixed with a coelenterazine solution.

The luminescence signals increased with increasing the concentration of corticosterone and were strong enough to discriminate them from background luminescence.

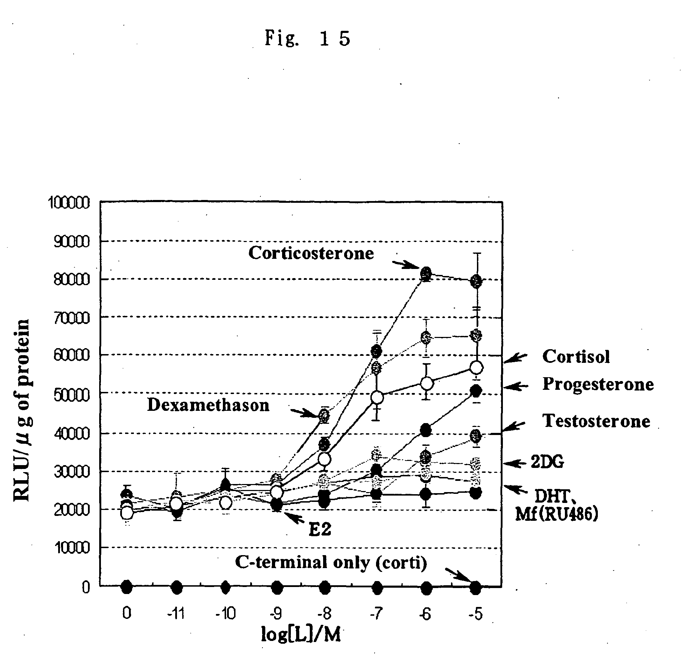

The GR translocation into the nucleus with several synthetic chemicals was confirmed using the pair of probes of this invention. As shown in Fig. 15, dexamethasone, progesterone and cortisol were found to induce the GR translocation into the nucleus.

### (3) GR translocation into the nucleus in living mice

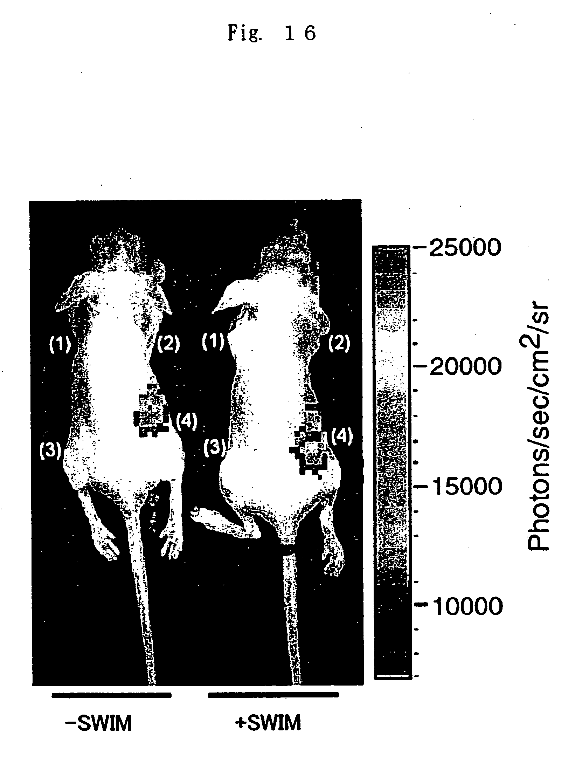

Next, it was verified that GR translocation into the nucleus within living mice could be observed using the pair of probes of this invention. NIH3T3 cells cotransfected with PcRDn-NLS and pcDRc-GR were implanted into the coria on each sites of the backs of living mice (Fig. 16). Twelve hours later, the mice were forced to swim in a water bath for 10 minutes to exert stress thereon. One hour later, coelenterazine was administered, and the luminescence intensities were measured with a CCD camera.

As shown in Fig. 16, the significant increase in luminescence signal could be observed from the stressed-mouse ("swimming (+)" in Fig. 16) relative to the unstressed-mouse ("swimming (-) in Fig. 16).

The results demonstrate that the physiological increase in corticosterone concentration of the living mice by stress stimulation can be detected with high accuracy upon reconstitution of RLuc.

### <Example 8>

Translocation of Sterol Regulatory Element-Binding Proten-2 (SREBP-2) into the nucleus was examined using a pair of probes of this invention in the same manner as in Example 7.

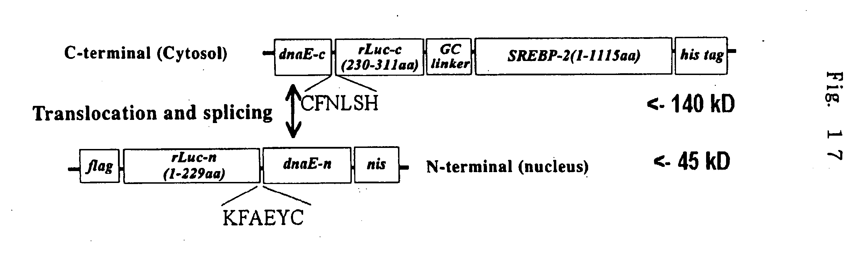

Plasmid pcDRc-AR in Examples 1 to 6 was cleaved with restriction endonucleases NotI and XhoI to remove the cDNA encoding AR, and a cDNA encoding SREBP-2 was inserted at NotI and XhoI sites thereby constructing pcDRc-SREBP (Fig. 17).

The cDNA-encoding N-terminal domain of Rluc (Rluc-N; 1~229 aa) was modified by PCR to introduce the peptide (KFAEYC: SEQ ID NO: 4) to the C terminus of Rluc-N, and the cDNA-encoding C-terminal domain of Rluc (Rluc-C; 230~311 aa) was modified by PCR to introduce the peptide (CFNLSH: SEQ ID NO: 9) to the N terminus of Rluc-C.

### (1) Confirmation of SERBP-2 localization

For confirming that SERBP-2 connected to the C-terminal domains of RLuc and DnaE is correctly localized in intracellular organelles in animal cells, COS-7 cells were transfected with pcDRc-SREBP expression vector.

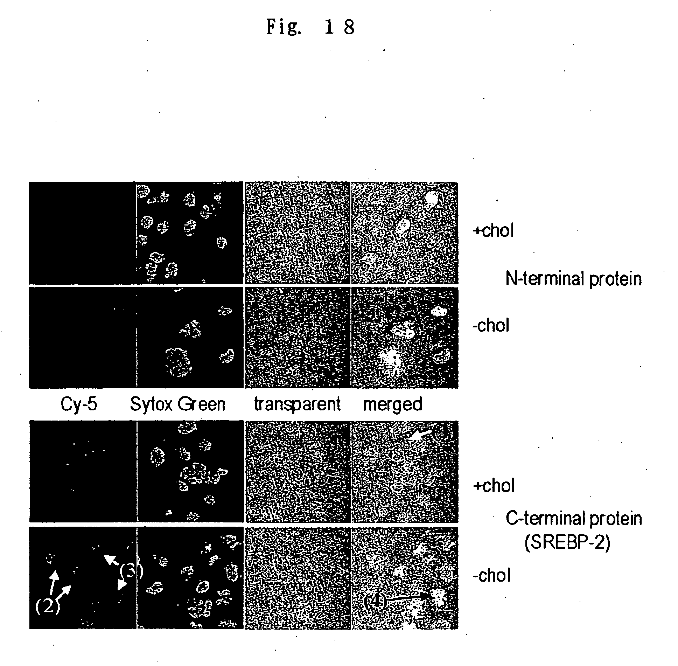

In the presence of cholesterol (Fig. 18, "+chol"), the probe with SREBP-2 was predominantly in cytoplasmic (Fig. 18(1)). On the other hand, in the absence of cholesterol (Fig. 18, "-chol") resulted in the nuclear localization of the probe. (Fig. 18(2)-(4)).

### (2) Quantification of SERBP-2 translocation into the nucleus

Translocation of SERPB-2 into the nucleus was qualified in the same manner as in Example 7. COS-7 cells were cotransfected with pcRDn-NLS and pcDRc-SREBP, and different concentrations of cholesterol were added to each microtiter well. The cells were harvested, and their lysates were mixed with a coelenterazine solution.

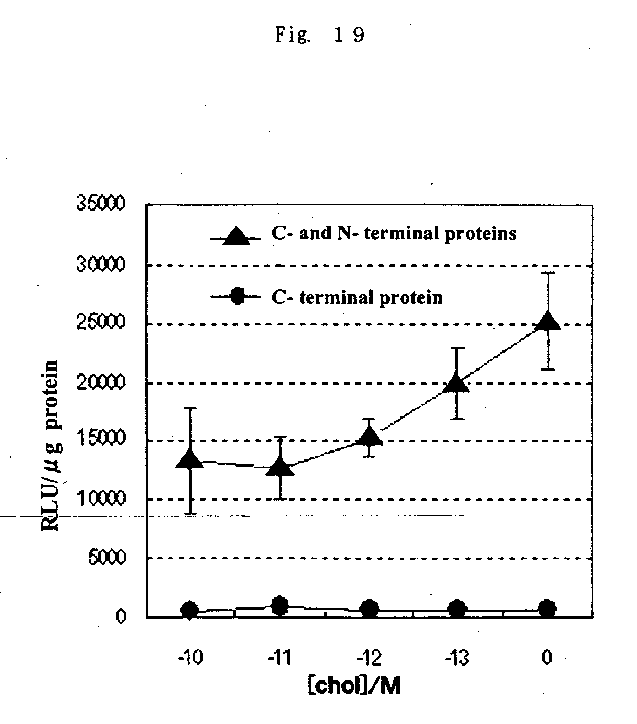

The luminescence signals increased with decreasing the concentration of cholesterol (Fig. 19) and were strong enough to discriminate them from background luminescence. These results indicate that the extent of SREBP-2 translocation into the nucleus can be detected using the luminescence intensity as an index.

### <Example 9>

Translocation of Signal Transducer and Activator of Transcription 3 (STAT-3) into the nucleus was examined using a pair of probes of this invention in the same manner as in Examples 7 and 8.

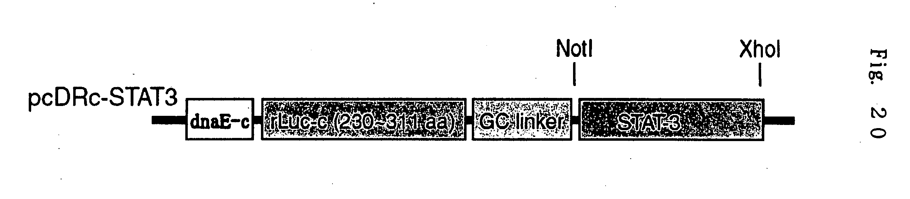

Plasmid pcDRc-AR in Examples 1 to 6 was cleaved with restriction endonucleases NotI and XhoI to remove the cDNA encoding AR, and a cDNA encoding STAT-3 was inserted at NotI and XhoI sites thereby constructing pcDRc- STAT-3 (Fig. 20).

### (1) Confirmation of STAT-3 localization

For confirming that STAT-3 connected to the C-terminal domains of RLuc and DnaE is correctly localized in intracellular organelles in animal cells, HEK293 cells were transfected with pcDRc-STAT3 expression vector.

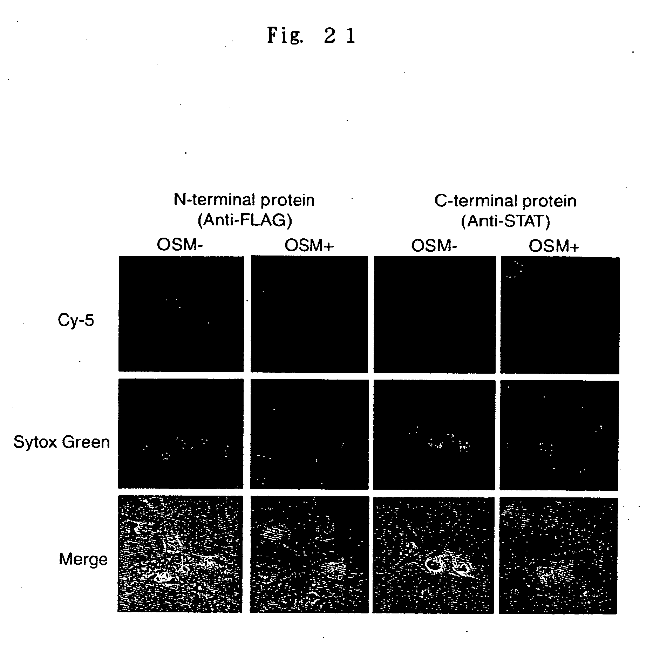

In the absence of oncostatin M (OSM), a ligand, the probe with STAT3 was predominantly in cytoplasmic (Fig. 21, "OSM-"), whereas addition of OSM resulted in the nuclear localization of the probe (Fig. 21, "OSM+").

### (2) Quantification of translocation of STAT-3 into the nucleus

Translocation of STAT-3 into the nucleus was qualified in the same manner as in Examples 7 and 8.

HEK293 cells were cotransfected with pcRDn-NLS and pcDRc-STAT3, and different concentrations of OSM were added to each microtiter well. The cells were harvested, and their lysates were mixed with a coelenterazine solution.

The luminescence signals increased with decreasing the concentration of cOSM (Fig. 21) and were strong enough to discriminate them from background luminescence. These results indicate that the extent of STAT-3 translocation into the nucleus can be detected using the luminescence intensity as an index.

### Industrial Applicability

As has been thus far described in detail, this invention provides a method for non-invasive imaging of protein nuclear transport in living cells or non-human animals. Further, this invention provides a method for quantifying the extent of protein translocation into the nucleus conveniently. The method for detecting and quantifying protein nuclear transport using the pair of probes of this invention is, unlike ordinary fluorescence techniques, free of background signals as a bioluminescence method, and makes it possible to perform the detection with high accuracy and high sensitivity. Accordingly, its application is expected to therapy, identification of toxics, development of drugs and the like.

The pair of probes for detecting protein nuclear transport comprises Probe I in which a protein whose nuclear transport is to be detected or quantified is connected to an N-terminal end or a C-terminal end of a fusion protein [intein-C/reporter protein-C] wherein at least a C-terminal side polypeptide of an intein and a C-terminal side polypeptide of a reporter protein are connected in this order, and Probe II in which a nuclear localization signal is connected to an N-terminal end or a C-terminal end of a fusion protein [reporter protein-N/intein-N] wherein at least the remaining N-terminal side polypeptide of the reporter protein and the remaining N-terminal side polypeptide of the intein are connected in this order.

Likewise, the pair of probes for detecting protein nuclear transport comprises Probe I in which a protein whose nuclear transport is to be detected or quantified is connected to an N-terminal end or a C-terminal end of a fusion protein [reporter protein-N/intein-N] wherein at least a N-terminal side polypeptide of a reporter protein and a N-terminal side polypeptide of an intein are connected in this order, and Probe II in which a nuclear localization signal is connected to an N-terminal end or a C-terminal end of a fusion protein [intein-C/reporter protein-C] wherein at least the remaining C-terminal side polypeptide of the intein and the remaining C-terminal side polypeptide of the reporter protein are connected in this order.

When these pairs of probes are introduced into cells, Probe I resides in the cytosol, and Probe II is localized in the nucleus. When the protein in Probe I recognizes and binds to a bioactive substance, Probe I translocates from the cytosol into the nucleus, and approaches Probe II localized in the nucleus. Upon the approach, the divided inteins in Probe I and Probe II are cut out by splicing, thereby the reporter protein is reconstituted. Therefore, the protein translocation into the nucleus induced by the bioactive substance can be detected with high accuracy with a measurement of the signal of the reporter protein.

In the pair of probes for detecting protein nuclear transport, the intein is DnaE derived from blue-green algae, so that the intein is surely cut out automatically.

In the pair of probes for detecting protein nuclear transport, the reporter protein is luciferase, a luminous catalytic enzyme. When luciferase is reconstituted upon occurrence of splicing between Probe I and Probe II, the active center is formed to emit light easily be detectable with a luminometer.

The method for detecting and quantifying protein nuclear transport in the invention comprises causing Probe I of the pair of probes and the bioactive substance to coexist in the cytosol, localizing Probe II in the nucleus, and measuring a signal of the reporter protein within the nucleus. When the protein recognizes and binds to the bioactive substance, Probe I translocates from the cytosol into the nucleus. Then, Probe I approaches Probe II localized in the nucleus, the divided inteins in Probe I and Probe II are cut out by splicing to reconstitute the reporter protein. Accordingly, by the signal of the reporter protein, the protein translocation into the nucleus induced by the bioactive substance can be detected surely with high accuracy. It is also possible to quantify the protein translocation into the nucleus.

In the method for detecting and quantifying protein nuclear transport in the invention, polynucleotides expressing the pair of probes are introduced into a cell, whereby it is possible that Probe I and the bioactive substance are coexisted in the cytosol and Probe II is localized in the nucleus. In the method for detecting and quantifying protein nuclear transport in the seventh invention, polynucleotides expressing the pair of probes are introduced into a non-human animal multipotent cell and the cell is subjected to ontogenesis, whereby it is possible that Probe I and the bioactive substance are coexisted in the cytosol and Probe II is localized in the nucleus in all cells of this animal or its progeny.

The method for screening a nuclear transport -inducing substance in the invention comprises introducing Probe I of the pair of probes into the cytosol, localizing Probe II in the nucleus, introducing a nuclear transport-inducing candidate substance into the cytosol, and measuring a signal of the reporter protein in the nucleus. Binding of the candidate substance with the protein in Probe I induces the protein translocation into the nucleus, Probe I approaches Probe II localized in the nucleus, and the divided inteins in Probe I and Probe II are cut out by splicing, thereby the reporter protein is reconstituted. Accordingly, by measuring the signal of the reporter protein, it can be judged with high accuracy whether or not the candidate substance acts on the protein as the nuclear transport -inducing substance.

The method for screening a nuclear transport-inhibiting substance in the invention comprises introducing Probe I of the pair of probes into the cytosol, localizing Probe II in the nucleus, introducing a nuclear transport-inhibiting candidate substance into the cytosol, further introducing a nuclear transport-inducing substance into the cytosol, measuring a signal of the reporter protein in the nucleus, and comparing the signal with a signal of the reporter protein obtained by introducing only the protein nuclear transport-inducing substance into the cytosol. In the case of the candidate substance acting as the nuclear transport -inhibiting substance, the candidate substance first binds to the protein, and hence the nuclear transport-inducing substance cannot bind to the protein. Consequently, the signal of the reporter protein is reduced in comparison to the signal of the reporter protein in introducing the nuclear transport-inducing substance only into the cytosol. On the other hand, where the candidate substance does not act as the nuclear transport-inhibiting substance, the candidate substance does not bind to the protein, and therefore the nuclear transport-inducing substance in the cytosol binds to the protein, which leads translocation of the protein into the nucleus. As a result, the signal of the reporter protein becomes equal when only the nuclear transport-inducing substance is introduced into the cytosol.

In the screening method in the invention, polynucleotides expressing the pair of probes are introduced into the cell for introducing Probe I into the cytosol and localizing Probe II in the nucleus. In the screening method in the invention, polynucleotides expressing the pair of probes are introduced into a non-human animal multipotent cell and the cell is subjected to ontogenesis, whereby it is possible that the Probe I is introduced in the cytosol and Probe II is localized in the nucleus in all cells of this animal or its progeny.

## Claims

1. A method for detecting and quantifying protein nuclear transport induced by action of a bioactive substance, which comprises
introducing a pair of probes and the bioactive substance into a cell, wherein the cell is neither a human embryonic stem cell, nor a cell which forms part of a human being, wherein said pair of probes comprises
Probe I in which a protein whose nuclear transport is to be detected or quantified is connected to an N-terminal end or a C-terminal end of a fusion protein [intein-C/reporter protein-C] wherein at least a C-terminal side polypeptide of an intein and a C-terminal side polypeptide of a reporter protein are connected in this order, and
Probe II in which a nuclear localization signal is connected to an N-terminal end or a C-terminal end of a fusion protein [reporter protein -N/intein-N] wherein at least the remaining N-terminal side polypeptide of the reporter protein and the remaining N-terminal side polypeptide of the intein are connected in this order; or
Probe I in which a protein whose nuclear transport is to be detected or quantified is connected to an N-terminal end or a C-terminal end of a fusion protein [reporter protein-N/intein-N] wherein at least a N-terminal side polypeptide of a reporter protein and a N-terminal side polypeptide of an intein are connected in this order, and Probe II in which a nuclear localization signal is connected to an N-terminal end or a C-terminal end of a fusion protein [intein-C/ reporter protein-C] wherein at least the remaining C-terminal side polypeptide of the intein and the remaining C-terminal side polypeptide of the reporter protein are connected in this order,
thereby making Probe I and the bioactive substance coexist in the cytosol, and
localizing Probe II in the nucleus, and
measuring a signal of the reporter protein within the nucleus.

2. The method of claim 1, wherein the intein is a DnaE intein derived from blue-green algae and/or the reporter protein is luciferase.

3. The detecting and quantifying method of claim 1, wherein polynucleotides expressing a pair of probes as defined in claim 1 or claim 2 are introduced into a cell thereby making Probe I and the bioactive substance coexist in the cytosol and localizing Probe II in the nucleus.

4. The detecting and quantifying method of claim 1, wherein polynucleotides expressing a pair of probes as defined in claim 1 or claim 2 are introduced into a non-human animal multipotent cell and the cell is subjected to ontogenesis thereby making Probe I and the bioactive substance coexist in the cytosol and localizing Probe II in the nucleus in all cells of the animal or its progeny.

5. A method for screening a protein nuclear transport-inducing substance, which comprises
introducing a pair of probes as defined in claim 1 or claim 2 and a nuclear transport-inducing candidate substance into a cell, thereby making Probe I and the candidate substance coexist in the cytosol, and
localizing Probe II in the nucleus, and
measuring a signal of the reporter protein in the nucleus, wherein the cell is neither a human embryonic stem cell, nor a cell which forms part of a human being.

6. A method for screening a protein nuclear transport-inhibiting substance, which comprises
introducing a pair of probes as defined in claims 1 or 2 and a nuclear transport-inhibiting candidate substance into a cell, thereby making Probe I and the substance coexist in the cytosol and localizing Probe II in the nucleus,
further introducing a nuclear transport-inducing substance into the cytosol,
measuring a signal of the reporter protein in the nucleus, and comparing the signal with a signal of the reporter protein obtained by introducing only the protein nuclear transport-inducing substance into the cytosol, wherein the cell is neither a human embryonic stem cell, nor a cell which forms part of a human being.

7. The screening method of claim 5 or 6, wherein polynucleotides expressing the pair of probes as defined in claim 1 or claim 2 are introduced into a cell thereby introducing Probe I into the cytosol and localizing Probe II in the nucleus.

8. The screening method of claim 5 or 6, wherein polynucleotides expressing the pair of probes as defined in claim 1 or claim 2 are introduced into a non-human animal multipotent cell and the cell is subjected to ontogenesis thereby introducing Probe I in the cytosol and localizing Probe II in the nucleus in all cells of the animal or its progeny.

## Patentansprüche

1. verfahren zum Nachweisen und Quantifizieren des Kerntransports von Proteinen, der durch die Wirkung einer bioaktiven Substanz induziert wird, wobei man bei dem Verfahren
ein Sondenpaar und die bioaktive Substanz in eine Zelle einbringt, wobei die Zelle weder eine humane embryonale Stammzelle noch eine Zelle ist, die einen Teil eines Menschen ausmacht, wobei das Sondenpaar Folgendes umfasst:
Sonde I, in der ein Protein, dessen Kerntransport nachgewiesen oder quantifiziert werden soll, an einen N-Terminus oder einen C-Terminus eines Fusionsproteins [Intein-C/Reporterprotein-C] gebunden ist, wobei mindestens ein auf der C-terminalen Seite befindliches Polypeptid eines Inteins und ein auf der C-terminalen Seite befindliches Polypeptid eines Reporterproteins in dieser Reihenfolge verbunden sind, und
Sonde II, in der ein Kernlokalisationssignal an einen N-Terminus oder einem C-Terminus eines Fusionsproteins [Reporterprotein-N/Intein-N] gebunden ist, wobei mindestens das restliche auf der N-terminalen Seite befindliche Polypeptid des Reporterproteins und das restliche auf der N-terminalen Seite befindliche Polypeptid des Inteins in dieser Reihenfolge verbunden sind, oder
Sonde I, in der ein Protein, dessen Kerntransport nachgewiesen oder quantifiziert werden soll, an einen N-Terminus oder einem C-Terminus eines Fusionsproteins [Reporterprotein-N/Intein-N] gebunden ist, wobei mindestens ein auf der N-terminalen Seite befindliches Polypeptid eines Reporterproteins und ein auf der N-terminalen Seite befindliches Polypeptid eines Inteins in dieser Reihenfolge verbunden sind, und
Sonde II, in der ein Kernlokalisationssignal an einen N-Terminus oder einem C-Terminus eines Fusionsproteins [Intein-C/Reporterprotein-C] gebunden ist, wobei mindestens das restliche Polypeptid auf der C-terminalen Seite des Inteins und das restliche Polypeptid auf der C-terminalen Seite des Reporterproteins in dieser Reihenfolge verbunden sind,
wodurch man die Sonde I und die bioaktive Substanz im Zytosol koexistieren lässt und
Sonde II im Kern lokalisiert, und
ein Signal des Reporterproteins im Kern misst.

2. Verfahren nach Anspruch 1, wobei es sich bei dem Intein um ein aus blaugrünen Algen stammendes DnaE-Intein handelt und/oder das Reporterprotein Luciferase ist.

3. verfahren zum Nachweisen und Quantifizieren nach Anspruch 1, wobei Polynukleotide, die ein Sondenpaar nach Anspruch 1 oder Anspruch 2 exprimieren, in eine Zelle eingebracht werden, wodurch man die Sonde I und die bioaktive Substanz im Zytosol koexistieren lässt und Sonde II im Kern lokalisiert.

4. Verfahren zum Nachweisen und Quantifizieren nach Anspruch 1, wobei Polynukleotide, die ein Sondenpaar nach Anspruch 1 oder Anspruch 2 exprimieren, in eine multipotente Zelle eines nicht-menschlichen Tiers eingebracht werden und die zelle einer Ontogenese unterworden wird, wodurch man in allen Zellen des Tiers oder seiner Nachkommenschaft die Sonde I und die bioaktive Substanz im Zytosol koexistieren lässt und Sonde II im Kern lokalisiert.

5. Verfahren zum Screening einer den Kerntransport eines Proteins induzierenden Substanz, wobei man bei dem Verfahren
ein Sondenpaar nach Anspruch 1 oder Anspruch 2 und eine den Kerntransport induzierende Kandidatensubstanz in eine zelle einbringt, wodurch man die Sonde I und die Kandidatensubstanz im zytosol koexistieren lässt und Sonde II im Kern lokalisiert, und
ein Signal des Reporterproteins im Kern misst, wobei die Zelle weder eine humane embryonale Stammzelle noch eine Zelle ist, die einen Teil eines Menschen ausmacht.

6. Verfahren zum Screening einer den Kerntransport eines Proteins inhibierenden Substanz, wobei man bei dem Verfahren
ein Sondenpaar nach Anspruch 1 oder Anspruch 2 und eine den Kerntransport inhibierende Kandidatensubstanz in eine Zelle einbringt, wodurch man die Sonde I und die Substanz im Zytosol koexistieren lässt und Sonde II im Kern lokalisiert, und
weiterhin eine den Kerntransport induzierende Substanz in das Zytosol einbringt,
ein Signal des Reporterproteins im Kern misst und das Signal mit einem Signal des Reporterproteins vergleicht, das erhalten wird, indem man nur die den Kerntransport eines Proteins induzierende Substanz in das Zytosol einbringt, wobei die Zelle weder eine humane embryonale Stammzelle noch eine Zelle ist, die einen Teil eines Menschen ausmacht.

7. Screeningverfahren nach Anspruch 5 oder 6, wobei Polynukleotide, die das Sondenpaar nach Anspruch 1 oder Anspruch 2 exprimieren, in eine zelle eingebracht werden, wodurch man die Sonde I in das Zytosol einbringt und Sonde II im Kern lokalisiert.

8. Screeningverfahren nach Anspruch 5 oder 6, wobei Polynukleotide, die das Sondenpaar nach Anspruch 1 oder Anspruch 2 exprimieren, in eine multipotente Zelle eines nicht-menschlichen Tiers eingebracht werden und die zelle einer Ontogenese unterworden wird, wodurch man in allen Zellen des Tiers oder seiner Nachkommenschaft die Sonde I in das Zytosol einbringt und Sonde II im Kern lokalisiert.

## Revendications

1. Procédé pour détecter et quantifier un transport nucléaire des protéines, induit par l'action d'une substance bioactive, qui comprend :
l'introduction d'une paire de sondes et de la substance bioactive dans une cellule, la cellule n'étant ni une cellule souche embryonnaire humaine, ni une cellule formant une partie d'un être humain, ladite paire de sondes comprenant :
une sonde I, dans laquelle une protéine dont le transport nucléaire doit être détecté ou quantifié est reliée à une extrémité N-terminale ou à une extrémité C-terminale d'une protéine de fusion [intéine-C/protéine rapporteuse-C], au moins un polypeptide du côté C-terminal d'une intéine et un polypeptide du côté C-terminal d'une protéine rapporteuse étant reliés dans cet ordre, et
une sonde II, dans laquelle un signal de localisation nucléaire est relié à une extrémité N-terminale ou à une extrémité C-terminale d'une protéine de fusion [protéine rapporteuse-N/intéine-N], au moins le polypeptide du côté N-terminal restant de la protéine rapporteuse et le polypeptide du côté N-terminal restant de l'intéine étant reliés dans cet ordre ; ou
une sonde I, dans laquelle une protéine dont le transport nucléaire doit être détecté ou quantifié est reliée à une extrémité N-terminale ou une extrémité C-terminale d'une protéine de fusion [protéine rapporteuse-N/intéine-N], au moins un polypeptide du côté N-terminal d'une protéine rapporteuse et un polypeptide du côté N-terminal d'une intéine étant reliés dans cet ordre, et
une sonde II, dans laquelle un signal de localisation nucléaire est relié à une extrémité N-terminale ou à une extrémité C-terminale d'une protéine de fusion [intéine-C/protéine rapporteuse-C], au moins le polypeptide du côté C-terminal restant de l'intéine et
le polypeptide du côté C-terminal restant de la protéine rapporteuse étant reliés dans cet ordre,
de façon à faire en sorte que la sonde I et la substance bioactive coexistent dans le cytosol, et
la localisation de la sonde II dans le noyau, et
la mesure d'un signal de la protéine rapporteuse à l'intérieur du noyau.

2. Procédé de la revendication 1, dans lequel l'intéine est une intéine DnaE, qui dérive de cyanobactéries et/ou la protéine rapporteuse est la luciférase.

3. Procédé de détection et de quantification de la revendication 1, dans lequel les polynucléotides exprimant une paire de sondes telles que définies dans la revendication 1 ou 2 sont introduits dans une cellule, de façon à faire en sorte que à sonde I et la substance bioactive coexistent dans le cytosol, et à localiser la sonde II dans le noyau.

4. Procédé de détection et de quantification de la revendication 1, dans lequel les polynucléotides exprimant une paire de sondes telles que définies dans la revendication 1 ou 2 sont introduits dans une cellule multipotente d'un animal non humain, et la cellule est soumise à une ontogenèse, de façon à faire en sorte que la sonde I et la substance bioactive coexistent dans le cytosol, et à localiser la sonde II dans le noyau dans toutes les cellules de l'animal ou de sa descendance.

5. Procédé pour le criblage d'une substance induisant le transport nucléaire des protéines, qui comprend :
l'introduction, dans une cellule, d'une paire de sondes telles que définies dans la revendication 1 ou 2, et
d'une substance candidate induisant le transport nucléaire, de façon à faire en sorte que la sonde I et
la substance candidate coexistent dans le cytosol, et
la localisation de la sonde II dans le noyau, et
la mesure d'un signal de la protéine rapporteuse dans le noyau, la cellule n'étant ni une cellule souche embryonnaire humaine, ni une cellule qui fait partie d'un être humain.

6. Procédé pour cribler une substance inhibant le transport nucléaire des protéines, qui comprend :
l'introduction, dans une cellule, d'une paire de sondes telles que définies dans la revendication 1 ou 2, et d'une substance candidate inhibant le transport nucléaire, de façon à faire en sorte que la sonde I et la substance coexistent dans le cytosol, et à localiser la sonde II dans le noyau,
en outre, l'introduction, dans le cytosol, d'une substance induisant le transport nucléaire,
la mesure d'un signal de la protéine rapporteuse dans le noyau, et
la comparaison du signal à un signal de la protéine rapporteuse, obtenu par introduction, dans le cytosol, uniquement de la substance induisant le transport nucléaire des protéines, la cellule n'étant ni une cellule souche embryonnaire humaine, ni une cellule qui fait partie d'un être humain.

7. Procédé de criblage de la revendication 5 ou 6, dans lequel les polynucléotides exprimant la paire de sondes telles que définies dans la revendication 1 ou 2 sont introduits dans une cellule, de façon à introduire la sonde I dans le cytosol et à localiser la sonde II dans le noyau.

8. Procédé de criblage de la revendication 5 ou 6, dans lequel les polynucléotides exprimant la paire de sondes telles que définies dans la revendication 1 ou 2 est introduite dans une cellule multipotente d'un animal non humain, et la cellule est soumise à une ontogenèse, de façon à introduire la sonde I dans le cytosol et à localiser la sonde II dans le noyau, dans toutes les cellules de l'animal ou de sa descendance.


### SEQUENCE LISTING

<110> Japan Science and Technology Agency
<120> A Pair of Probes for Detect ing Protein Nuclear Translocation
<130> 04F057PCT
<150> JP 2004-066424
   <151> 2004-03-09
<160> 9
<170> Patent In version 3.1
<210> 1
   <211> 24
   <212> PRT
   <213> Simian virus 40
<400> 1
   Asp Pro Lys Lys Lys Arg Lys Val Asp Pro Lys Lys Lys Arg Lys Val
   1 5 10 15
   Asp Pro Lys Lys Lys Arg Lys Val
   20
<210> 2
   <211> 16
   <212> PRT
   <213> Xenopus laevis
<400> 2
   Lys Arg Pro Ala Ala Ile Lys Lys Ala Gly Gln Ala Lys Lys Lys Lys
   1 5 10 15
<210> 3
   <211> 15
   <212> PRT
   <213> Human immunodeficiency virus type 1
<400> 3
   Arg Gln Ala Arg Arg Asn Arg Arg Asn Arg Arg Arg Arg Gln Arg
   1 5 10 15
<210> 4
   <211> 6
   <212> PRT
   <213> Artificial
<220>
   <223> synthesized oligopeptide
<400> 4
   Lys Phe Ala Glu Tyr Cys
   1 5
<210> 5
   <211> 8
   <212> PRT
   <213> Artificial
<220>
   <223> synthesized oligopeptide
<400> 5
   Asp Tyr Lys Asp Asp Asp Asp Lys
   1 5
<210> 6
   <211> 5
   <212> PRT
   <213> Artificial
<220>
   <223> Synthesized oligopeptide
<400> 6
   Phe Asn Leu Ser His
   1 5
<210> 7
   <211> 6
   <212> PRT
   <213> Artificial
<220>
   <223> synthesized oligopeptide
<400> 7
   Gly Gly Gly Gly Ser Gly
   1 5
<210> 8
   <211> 5
   <212> PRT
   <213> Artificial
<220>
   <223> synthesized oligopeptide
<400> 8
   Lys Phe Ala Glu Tyr
   1 5
<210> 9
   <211> 6
   <212> PRT
   <213> Artificial
<220>
   <223> synthesized oligopeptide
<400> 9
   Cys Phe Asn Leu Ser His
   1 5